# EUROPEAN PATENT SPECIFICATION

(11) **EP 0 546 055 B1**
(45) Date of publication and mention of the grant of the patent: **10.07.1996**
(21) Application number: 91916082.0
(22) Date of filing: 27.08.1991
(51) Int. Cl.: C08F 8/00, C07K 1/04

(54) **POLYETHYLENE GLYCOL DERIVATIVES FOR SOLID-PHASE APPLICATIONS**
POLYÄTHYLENGLYKOLDERIVATE ZUR FESTPHASEANWENDUNGEN
DERIVES DE POLYETHYLENE-GLYCOL DESTINES A DES APPLICATIONS EN PHASE SOLIDE

(30) Priority: 31.08.1990 US 576314; 14.06.1991 US 715289
(43) Date of publication of application: 16.06.1993
(60) Divisional application: 95112933.7
(73) Proprietor: REGENTS OF THE UNIVERSITY OF MINNESOTA, Minneapolis, Minnesota 55415-1226 (US)
(72) Inventor: BARANY, George, Falcon Heights, MN 55113 (US); ALBERICIO, Fernando, E-08028 Barcelona (ES); CHANG, Jane, Vernon Hills, IL 60061 (US); ZALIPSKY, Samuel, Princeton, NJ 08540 (US); SOLE, Nuria, A., Minneapolis, MN 55414 (US)
(74) Representative: Holdcroft, James Gerald, Dr.
(86) International application number: US9106103
(87) International publication number: WO9204384

(56) References cited:
- EP-A- 0 187 391
- STN International, File CA, Chemical Abstracts, volume 114, no. 25, 24 June 1991, (Columbus, Ohio, US), Pillai, V.N. Rajasekharan et al: "Synthesis of thioredoxin partial sequences on a polyethylene- glycol-grafted polystyrene support with a photoly- tically detachable 2-nitrobenzyl anchoring group", abstract 247733r, & Indian J. Chem., Sect. B, 30B(2), 205-12
- STN International, File CA, Chemical Abstracts, volume 106, no. 9, 2 March 1987, (Columbus, Ohio, US), Zalipsky et al.: "Preparation and use of an aminoethyl polyethylene", abstract 67651p
- MAKROMOL.CHEM., Vol. 184, 1983 Heidi Hellermann et al: "Poly(ethylene glycol)s Grafted onto Crosslinked Polystyrenes,2" pp. 2603-2617
- MAKROMOL.CHEM.,RAPID COMMUN, Vol. 3, 1982 Heidi Becker et al: "Polyethyleneglycols Grafted onto Crosslinked Polystyrenes: A New Class of Hydrophilic Polymeric Supports for Peptide Synthesis ", pp. 217-223

## Description

### Background of the Invention

Spacer arms are essential in many areas of modern biochemistry. Spacer arms can be defined as molecules which link one molecule to another molecule or to an inert support. Polyethylene glycol, for example, has been used advantageously to link enzymes to insoluble carriers and other biomolecules while retaining the activity of the enzyme. M. Stark and K. Holmberg, Biotech. and Bioeng., 34:942-950 (1989). This concept has important consequences for industrial processes using immobilized enzymes (e.g., affinity column purification processes), and for diagnostic assays (e.g., ELISA (Enzyme linked immuno sorbant assay) assays). Two other areas in which polyethylene glycol spacer arms have been used are peptide synthesis and sequencing. The coupling rate of protected nucleotide and amino acid residues to inert supports, such as silica, membrane, and polystyrene supports, often increases commensurately with the separation of the reaction site from the support backbone. Similar effects have been shown for sequencing of solid-phase immobilized samples. J.K. Inman et al., In Solid Phase Methods in Protein Sequence Analysis, Previero and Coletti-Previero, (Eds.), Elsevier, North-Holland Biomed. Press, pp. 81-94 (1977).

The effectiveness of solid-phase nucleic acid or peptide synthesis or sequence analysis is affected by the solid-phase or support which anchors the reactive sites. Polystyrene gels or porous glass have both been utilized as solid supports for peptide sequencing, for example. In many applications, the solvents used in the process can cause the polystyrene particles to change in volume, which causes blocking of the reaction column and back pressure. Conversely, porous glass is completely rigid and does not change in volume, but the chemical properties of porous glass derivatives have lacked reproducibility. Polymer particles, such as polystyrene particles, which have been derivatized so that reactive groups can be attached to them, have proved useful in many applications. Polyethylene glycol (PEG) structures have been used as chemically inert spacer arms because they are compatible with a wide range of solvents. Inman et al., ibid. The use of PEG spacer arms minimizes the steric effects caused by the support. PEG spacer arms provide another useful function in modifying the character of the pore space so that the support-bound reactive moiety is compatible with a wider range of solvents and reagents.

PEG-modified polystyrene (PEG-PS) resins have been described for use in solid-phase peptide sequencing. Inman et al., ibid. PEG-PS resins have also been utilized as phase transfer catalysts. W.M. McKenzie et al., J. Chem. Soc. Chem. Commun., p. 541-543 (1978); S.L. Regen et al., J. Amer. Chem. Soc., 101:116-120 (1979); J.G. Heffernan et al., J. Chem. Soc. Perkin II, p. 514-517 (1981); Y. Kimura et al., J. Org. Chem., 48:385-386 (1983); M. Tomoi et al., Reactive Polymers, 10:27-36 (1989). PEG-PS resins have been described as supports for solid-phase peptide synthesis. Becker et al., Makromol. Chem. Rapid Commun., 3:217-223 (1982); H. Hellermann, et al., Makromol. Chem., 184:2603-2617 (1983). However, PEG-PS resins prepared by the referenced methods suffer from several drawbacks. The reactions proceeded poorly with high molecular weight PEG (e.g., greater than 400 daltons) and symmetrical bifunctional PEG tended to form crosslinks. These problems were reduced by the anionic polymerization of ethylene oxide directly onto crosslinked polystyrene. Bayer et al., In Peptides: Structure and Function, V.J. Hruby and D.H. Rich (eds.), Proc. 8th Am. Peptide Symp. pp. 87-90, Pierce Chem. Co., Rockford, IL (1983). Bayer and Rapp, German Patent DE 3500180 Al (1986). However, the PEG chain lengths are difficult to control using this method, and the uniformity of the PEG polymers is uncertain. Another problem with this process is that the polystyrene is functionalized using chloromethyl ether, which is highly toxic, and residual chloromethyl groups can cause side reactions during peptide synthesis.

Another method of making PEG graft copolymers is described by Zalipsky et al., In Peptides:Structure and Function, C.M. Deber, V.J. Hruby and K.D. Kopple (eds.), Proc. 9th Am. Pep. Symp., pp. 257-260, Pierce Chem. Co., Rockford, IL (1985). In this method, certain heterobifunctional PEG derivatives of defined molecular weight (i.e., 2000 to 4000 daltons) were used. However, these derivatives are not readily available, which hinders their commercialization.

A method of preparing non-toxic and efficient solid supports which can be used with a wide range of solvents would be valuable for use in solid-phase synthesis or sequencing of peptides or nucleic acids, or for other solid-phase applications.

### Summary of the Invention

The present invention pertains to polyethylene glycol derivatized graft supports.

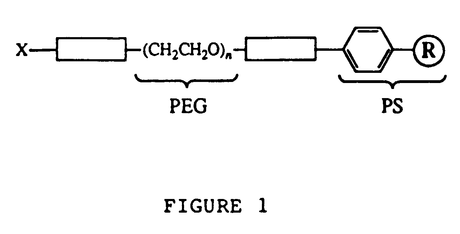

This specification discloses methods for making the supports, and methods of using the supports to synthesize peptides by solid-phase synthesis techniques. The PEG-graft supports of this invention comprise functionalized PEG derivatives which are covalently attached to solid supports. The PEG graft supports are represented by Formula I and shown in Figure 1: 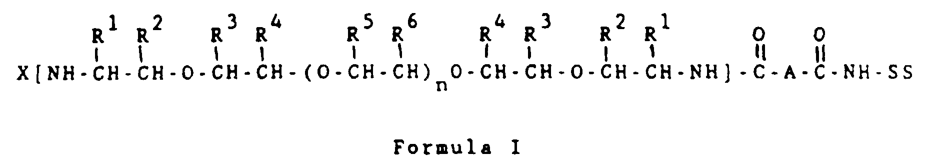 wherein n is an integer from 5 to 150; R¹, R², R³, R⁴, R⁵ and R⁶ are independently selected from the group consisting of hydrogen (H), and alkyl or aryl groups such as methyl, ethyl or phenyl; X is H or H₂N-B-NH-C(O)-A-C(O)-; and A and B are independently a straight chain or branched alkyl group, such as ethylene, propylene, isopropylene, butylene, isobutylene or other group up to C-10 in length (e.g., A derived from succinic, glutaric, adipic or other such acids; B derived from ethylenediamine or other aliphatic diamines), a CH-CH group (e.g., A derived from maleic acid) or an aromatic group (e.g., A derived from phthalic acid; B derived from phenylenediamine). SS represents the solid support. The terminal amino group can optionally be protected by N^{ω} protecting groups, such as tert-butyloxycarbonyl (Boc) and 9-fluorenylmethyloxycarbonyl (Fmoc) and other known protecting groups. The core portion of the formula, indicated within the brackets, corresponds to a series of readily available amino-functionalized polyethylene glycol (PEG) polymer derivatives.

The present invention provides compact, commercially viable routes for making functionalized inert supports for solid-phase applications using monofunctional or homobifunctional polyethylene glycol as the starting material. The present PEG graft supports provide advantageous physical and mechanical properties for solid-phase peptide synthesis, nucleic acid synthesis, and other applications where immobilized molecules are used.

### Brief Description of the Figures

Figure 1 shows the general structures of a polyethylene glycol-polystyrene (PEG-PS) graft supports. X illustrates the point from which biopolymer chain growth begins.

Figure 2 is a schematic showing a series of reactions leading to preparation of a spacer arm linker, its coupling to an amino-functionalized polystyrene resin, attachment of a handle to the resin-bound linker, and its use to synthesize a peptide.

Figure 3 is a schematic showing a series of reactions leading to further PEG-PS graft supports.

### Detailed Description of the Invention

The present invention pertains to resins comprising a functionalized polyethylene glycol derivatives covalently linked to a solid support. The resulting graft support comprises a symmetrical poly(oxyethylene) diamine derivative linked to the support and is represented by Formula I: 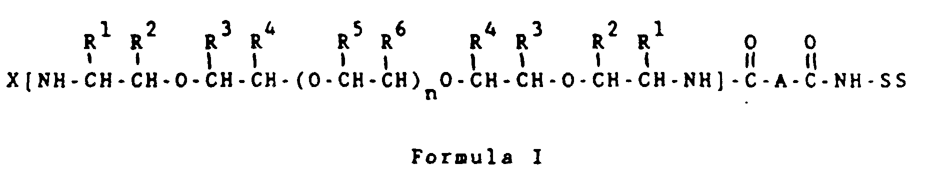 wherein n is an integer from 5 to 150; R¹, R², R³, R⁴, R⁵ and R⁶ are independently selected from the group consisting of hydrogen (H), and alkyl or aryl groups such as methyl, ethyl or phenyl; SS is a solid support; X is H or H₂N-B-NH-C(O)-A-C(O)-; and A and B are independently a straight chain or branched alkyl group, such as ethylene, propylene, isopropylene, butylene, isobutylene or the like up to C-10 in length (e.g., A derived from succinic, glutaric, adipic or other such acids; B derived from ethylenediamine or other aliphatic diamines); a CH-CH group (e.g., A derived from maleic acid) or an aromatic group (e.g., A derived from phthalic acid; B derived from phenylenediamine). The core portion of the formula, within the bracketed portion, corresponds to a series of polyoxyethylene diamine polymers. The amino group(s) can optionally be protected by known N^{ω} protecting groups.

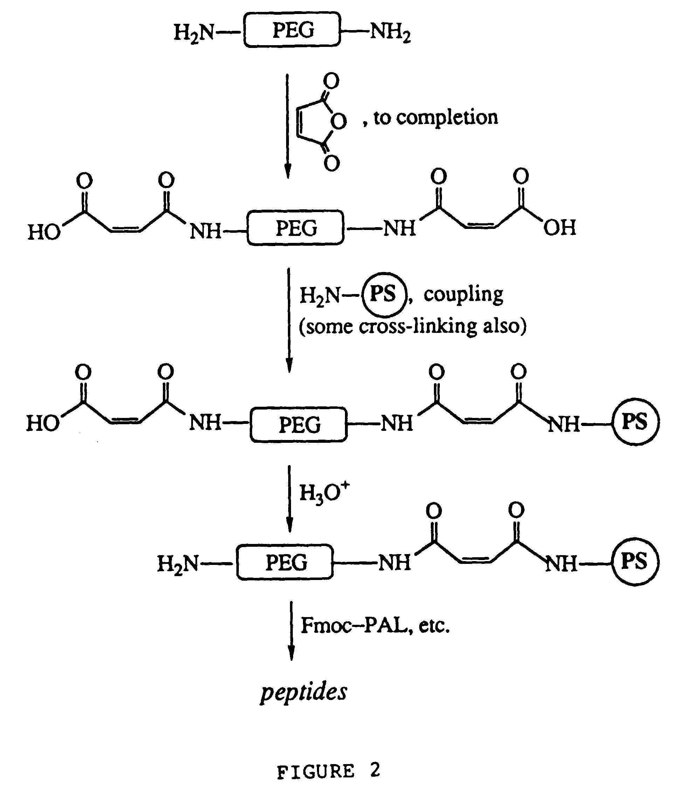

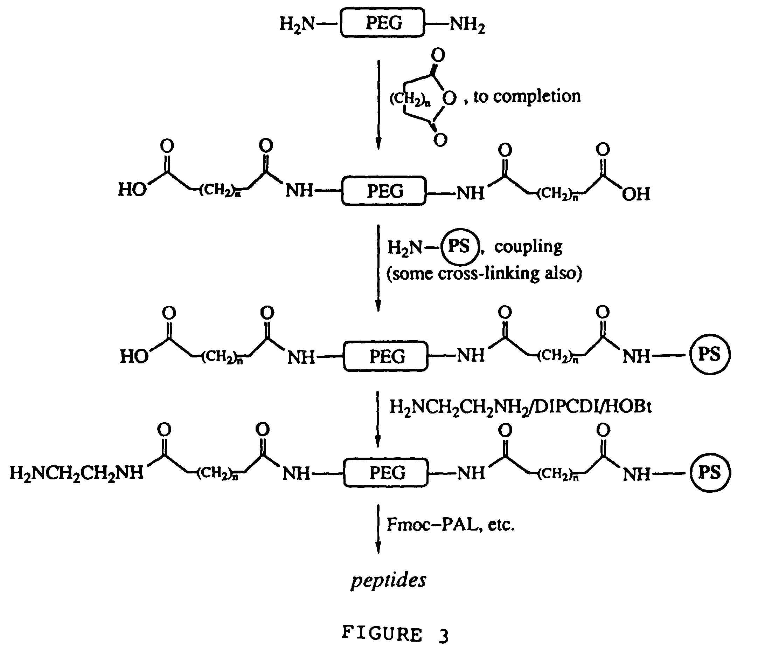

One method for producing the graft supports of Formula I is by reacting amino-functionalized core polymers with dicarboxylic acid derivatives, including anhydrides, to produce carboxyl-functional molecules. According to this method, the diamine polymer is reacted with at least two equivalents of the activated dicarboxylic acid derivative. Dicarboxylic acids which are useful in this method include alkyl diacids having up to about 12 carbon atoms, such as, for example, maleic, succinic, glutaric or adipic acid; anhydrides such as maleic, succinic or glutaric anhydride; or aromatic anhydrides, such as phthalic anhydride. In one embodiment of this method, the diamine polymer is reacted with succinic, maleic or glutaric anhydride to make representatives of the claimed compounds, bis(succinylated), bis(maleylated) or bis(glutarylated) PEG. Figures 2 and 3 illustrate the formation and subsequent coupling of these derivatives onto an amino-functionalized solid support.

Homobifunctional PEG diamine compounds which can be subsequently reacted to produce resins of Formula I have the general Formula III: 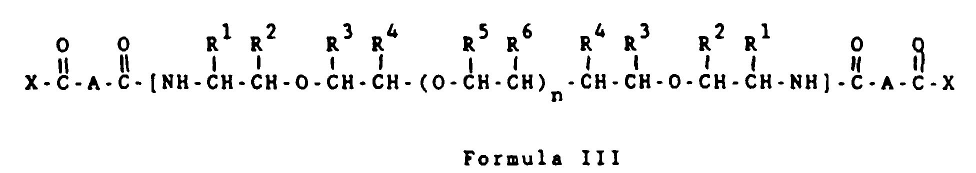 wherein n is an integer between 5 and 150; R¹, R², R³, R⁴, R⁵ and R⁶ are independently selected from the group consisting of H, alkyl groups and aryl groups; X is selected from the group consisting of OH, halogens and the activating group of an active ester or thioester; and A is selected from the group consisting of straight chain or branched alkyl groups having up to 10 carbon atoms, CH-CH groups and aromatic groups. The term "active ester" refers to compounds which activate carboxyl groups to make them undergo more ready reactions with amino groups. Activating groups which can be used in the present composition and method include, for example, trichlorophenyl (TCP) esters, pentafluorophenyl (PFP) esters, pentachlorophenyl (PCP) esters and methylphenylpyrazolinone (MPP) esters.

Symmetrical diamines which are used as the core portion of the polymer include polymers corresponding to the bracketed portion of the structures shown in Formula I and Formula III. Such PEG derivatives having a theoretical content of amino groups can be obtained by a number of known processes. See, for example, Duckmann et al., Makromol. Chem., 182:1379 (1983); Zalipsky et al., Eur. Polym. J., 19:1177 (1983). Polymers which are particularly useful for this purpose include a series of poly(oxyethylene) diamines having a molecular weight up to about 6000 daltons which are commercially available under the tradename Jeffamine® (Texaco Chemical Co., Bellaire, TX). The Jeffamine® poly(oxyethylene) diamine resins are aliphatic primary diamines structurally derived from polypropylene oxide-capped polyethylene glycol. These products are characterized by high total and primary amine contents. Other symmetrical diamines having the desired characteristics can be used. For some applications, symmetrical dicarboxylic acid-functionalized polymers having approximately the same general structure can be used.

Carboxyl-functionalized spacer arm linkers produced by the present method are then coupled to appropriate parent carriers which have been functionalized with amino groups. Carriers which are useful as solid phases in the present invention include macromolecules or solids, such as membranes, porous glass, silica, polystyrenes, polydimethylacrylamides, cotton or paper. Solid supports which are particularly useful include amino-functionalized polystyrene, aminomethyl polystyrene, aminoacyl polystyrene and p-methylbenzhydrylamine polystyrene. A particularly preferred support is an amino-functionalized polystyrene-co-1% divinylbenzene. Amino groups of the parent carrier can be covered by reacting one equivalent of the carrier based on the amino groups with an excess of the carboxyl-functionalized derivatives. Most of the amino groups on the carrier become substituted by the polyethylene glycol derivatives, thereby forming spacer arms having one free pendant carboxyl group.

Introduction of an amino functionality on the present PEG derivatives is desirable for many synthesis applications. This can be achieved by acid hydrolysis (see Figure 2) of the amide moiety, thereby exposing the amino group which was originally present on the diamine, or by further coupling of a free or monoprotected low molecular-weight diamine (e.g., ethylene or hexamethylene diamine) with the carboxylate end group (see Figure 3). For parent carriers, modified by bis(maleylated) PEG linkers (see Figure 2), for example, the terminal pendant maleyl group is selectively hydrolyzed by controlled treatment with an acid, e.g., trifluoroacetic acid or dilute hydrochloric acid (HCl), whereas the other maleyl group, now linking the PEG to the carrier, is essentially stable. By these methods, amino-functionalized PEG-modified materials are obtained rapidly, efficiently and economically.

Polyethylene glycol-polystyrene (PEG-PS) graft supports made by the methods of this invention are particularly useful. PEG-PS supports made using the present PEG derivatives have several desirable characteristics for solid-phase applications: they swell in a variety of solvents, are stable under the condition used in most solid-phase synthesis, and behave well in both batch and column reactors used in solid-phase applications, in particular, solid-phase peptide synthesis.

Solid-phase peptide synthesis typically begins with covalent attachment of the carboxyl end of a first amino acid to the solid support. The carboxyl group of an N^{α}-protected amino acid is covalently linked to a handle moiety which is attached to the amino group on the free end (the end not linked to the solid support) of the PEG spacer arm (Figures 1, 2 and 3). A "handle" is defined as a bifunctional spacer which serves to attach the initial amino acid residue to the polymeric support. One end of the handle incorporates a smoothly -cleavable protecting group and the other end of the handle couples to the functionalized solid support. Handles which can be used with the present spacer arms in solid-phase peptide synthesis include, for example acid-labile p-alkoxybenzyl (PAB) handles, photolabile o-nitrobenzyl ester handles, and handles such as those described by Albericio et al., J. Org. Chem., 55:3730-3743 (1990) and references cited therein.

The appropriate handles are coupled quantitatively in a single step onto the amino-functionalized supports to provide a general starting point of well-defined structures for peptide chain assembly. The handle protecting group is removed and the C-terminal residue of the N^{α}-protected first amino acid is coupled quantitatively to the handle. Once the handle is coupled to the solid-phase and the initial amino acid or peptide is attached to the handle, the general synthesis cycle proceeds. The synthesis cycle generally consists of deprotection of the N^{α}-amino group of the amino acid or peptide on the resin, washing, and, if necessary, a neutralization step, followed by reaction with a carboxyl-activated form of the next N^{α}-protected amino acid. The cycle is repeated to form the peptide or protein of interest. Solid-phase peptide synthesis methods using functionalized insoluble supports are well known. Merrifield, J. Am. Chem. Soc., 85:2149 (1963); Barany and Merrifield, In Peptides, Vol. 2, pp. 1-284 (1979); Barany et al., Int. J. Peptide Protein Res., 30:705-739 (1987).

The present PEG-derivatives are particularly useful as spacer arms, which separate the inert support from the reacting amino acids which are forming the peptide chain during the synthesis process. The choice of a spacer arm, which provides this distance, is a critical parameter in solid-phase applications.

In a preferred embodiment of the present invention, a variety of PEG spacer arms having an average molecular weight of about 2000 daltons were incorporated between amino functional groups on a polystyrene backbone and the point for attachment of the appropriate handles. The resultant PEG-PS graft supports contained approximately equal weight amounts PEG and PS. These supports showed reproducible advantage over PS supports with regard to physical and chemical properties such as swelling and in synthesis of model peptides. The PEG-PS supports of this invention allow peptide synthesis to be carried out using acetonitrile as the solvent for all reaction steps and washes. The control experiments using PS and employing acetonitrile as the solvent resulted in no peptide products.

In another embodiment, comparative experiments were carried out in which the difficult acyl carrier protein 65-74 decapeptide sequence was synthesized using Fmoc-amino acids. The peptide was produced in higher purity on this new PEG-PS than on either PS, Tentagel™ (a trademark of Rapp Chem., Tubingen, Germany), or Pepsyn K™ (a trademark of Cambridge Research Biochem., Cambridge, England). The PEG-PS material proved highly suitable both for flow-through synthesis and batch operation. Negligible back-pressures were found even at high flow rates.

The general usefulness of the PEG-PS graft support was demonstrated by syntheses of a number of large, (e.g., having about 30 to 60 residues) complex peptide sequences, such as cecropin analogues, calcitonin, β-endorphin, corticotropin releasing factor, two zinc finger binding sequences, and several partial sequences of HIV-1 tat protein. The improvements in synthetic efficiency which resulted from use of the present PEG-PS linkers appear to originate from one or more of the following: (i) a spacer arm effect removing the reaction sites from the vicinity of the polymer backbone; (ii) a general environmental effect which modifies the hydrophobic nature of the resin with a concomitantly favorable influence on reaction rates; and (iii) a specific effect on conformationally difficult couplings due to decreased secondary structure (hydrogen bond formation).

The PEG derivatives can also be used in nucleic acid synthesis or sequencing as spacer arms or linkers between an inert support and the reacting nucleotide or nucleic acid. For example, the derivatives can be attached to polystyrene resins as described above and used in amidite-mediated DNA synthesis. The PEG-PS resin swells in acetonitrile, which is used as a solvent in the amidite coupling method.

The invention will now be further illustrated by the following examples:

### EXAMPLES

### Example 1. Preparation of a Bis(maleylated)Derivative of Jeffamine® ED-2001

Maleic anhydride (4.5 g, 46 mmol) was added in one portion to a stirred solution of Jeffamine® ED-2001 (30 g, 15 mmol, 30 mmol amino functions) in tetrahydrofuran (90 mL) at 25°C. After 5 hours, the solvent was removed by evaporation in vacuo, and the resulting oil was added to cold stirred ether (100 mL) at 0°C over 5 minutes. The resulting white suspension was stirred at 0°C for another 15 minutes, then collected by filtration, rinsed thoroughly with ether, and dried over phosphorus pentoxide in vacuo. Yield: 28.9 g (88%). NMR data and elemental analysis were in accord with the expected structure shown below: 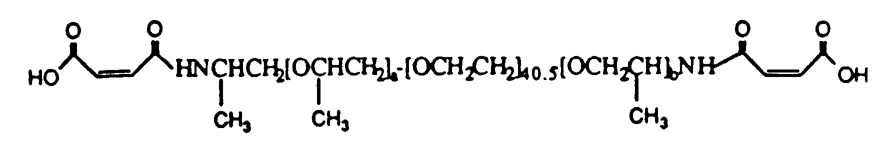 wherein a + b = 2.5.

### Example 2. Preparation of a Bis(gutarylated) Derivative of Jeffamine® ED-2001

A mixture of Jeffamine® ED-2001 (40 g, 20 mmol, 40 mmol amino functions) plus glutaric anhydride (5.0 g, 44 mmol) in dichloromethane (200 mL) was stirred at 25°C for 2 hours. The homogeneous reaction mixture, which gave a negative ninhydrin test indicating complete acylation, was concentrated in vacuo to provide a viscous oil which was decanted with vigorous stirring into a beaker containing ethyl ether (300 mL) at 4°C. A white precipitate formed quickly, which was collected, washed with further cold ether, and dried over phosphorus pentoxide in vacuo. Yield: 38 g (86%). NMR data and elemental analysis in accord with structure.

### Example 3. Procedure for Coupling PEG-Diacid Derivative to MBHA-Resin

To a stirred solution of the PEG-diacid produced in Example 1, (26 g, 11.8 mmol, 23.6 mmol carboxyl groups), in N,N-dimethylformamide (DMF) (30 mL), was added 1-hydroxybenzotriazole (HOBt; 0.81 g, 6.0 mmol) in DMF (3 mL) and benzotriazol-1-yl-oxy-tris-(dimethylamino)-phosphonium hexafluorophosphate (BOP; 2.63 g, 6.0 mmol) in DMF (8 mL). A solution of N,N-diisopropylethylamine (DIEA; 1.56 mL, 9 mmol) in dichloromethane (5 mL) was added over 10 minutes and the resulting solution stirred for another 10 minutes before transfer to a reaction vessel containing prewashed p-methylbenzhydrylamine (MBHA) polystyrene resin support (5.0 g, 0.6 mmol/g, 3.0 mmol amino groups). The mixture was shaken for 2 days, until a ninhydrin test on a sample was almost negative. Unreacted amino groups were capped by acetylation, and the support was then washed with dichloromethane for further treatment, as described in Example 7.

### Example 4. Procedure for Coupling PEG-Diacid Derivative to Nle-Resin

In a separate experiment, an aminomethyl polystyrene resin support (0.68 mmol/g) was derivatized with Fmoc-norleucine by standard procedures. A portion of the resultant 9-fluorenylmethyloxycarbonyl-Norleucine resin (Fmoc-Nle-resin)(0.23 g, 0.55 mmol/g, 0.13 mmol) was deprotected with piperidine-DMF (3:7, v/v) (2 + 10 min) and washed with DMF and dichloromethane. In the meanwhile, the PEG-diacid from Example 2 (0.86 g, 0.39 mmol, 0.78 mmol carboxyl groups), BOP (88 mg, 0.2 mmol) and HOBt (27 mg, 0.2 mmol) were dissolved in DMF (4 mL), and then a solution of DIEA (45 µL, 0.26 mmol) in dichloromethane (2 mL) was added. After a 10 min preactivation period at 25°C, the PEG solution was added to the deprotected and washed resin, and coupling was carried out at 25°C for 5 hours to give an essentially negative ninhydrin test. Capping was performed by the addition of acetic anhydride (40 µL, 0.4 mmol) in dichloromethane (2 mL) for 30 min. The final PEG-PS graft support (0.49 g) comprised PEG:PS:Nle = 0.51:0.45:0.04 based on elemental analysis and amino acid analysis, from which it can be calculated that about 40% of the PEG chains were involved in cross-linking.

### Example 5. Procedure for Removal of Terminal Maleyl Group

The PEG-functionalized support prepared in Example 4 was washed with trifluoroethanol on a sintered funnel, and transferred to a silanized round bottom flask. A mixture of trifluoroethanol/trifluoroacetic acid/water (8:1:1, 50 mL) was added and the suspension was heated at reflux (oil bath temp. 100°C) and stirred magnetically for 24 hours. The material was then washed with dichloromethane, 5% DIEA in dichloromethane and methanol, and dried in vacuo in P₂O₅. Amino group content was determined on an aliquot by picric acid titration, or by loading with an Fmoc-amino acid. The final PEG-PS comprises PEG:PS = 0.45:0.55 and has a loading of 0.17 mmol/g (based on CHN elemental analysis and amino acid analysis); it can be calculated that about 65% of the PEG chains in this support are acting as a spacer with the remainder involved in crosslinking.

### Example 6. Procedure for Ethylenediamine Addition

The PEG-PS support from Example 5 (0.4 g, 0.12 mmol original Norleucine (Nle) sites) was washed with dichloromethane for initial swelling, then N,N'-diisopropylcarbodiimide (DIPCDI) (94 µL, 0.6 mmol) and HOBt (81 mg, 0.6 mmol) in DMF (2 mL) were added to the resin for a 5 minute preactivation period. Ethylenediamine (40 µL, 0.6 mmol) in DMF (0.5 mL) was then added to the resin for a 5 hour reaction. Final washes with DMF, dichloromethane and methanol were performed. The resin (0.4 g; no weight change from this step) was dried in vacuo over P₂O₅, following which the usual loading measurements with 9-fluorenylmethyloxycarbonyl-Alanine (Fmoc-Ala)revealed a substitution level of 0.11 mmol/g, and Alanine-Norleucine) (Ala:Nle) ratio of 0.30 (consistent with earlier estimate of extent of cross-linking).

### Example 7. Batchwise Peptide Synthesis with Polyethylene Glycol-Polystyrene Graft (PEG-PS)

PEG-PS was prepared according to the procedure set out in Examples 4 and 7, starting with 5 grams of p-methylbenzhydrylamine resin (with loading 0.6 mmol/g), and 8.7 gram of PEG-PS product was obtained (loading 0.17 mmol/g). A 2 gram portion of this PEG-PS was extended with the Fmoc-PAL [5'-(4"-(9.fluorenylmethyloxycarbonyl) aminoethyl-3,5-dimethoxyphenoxy)valeric acid] handle which was linked by the BOP + HOBt protocol (D. Hudson, (1987) J. Org. Chem., 53:617-624) to give a totally functionalized ninhydrin negative product. A test peptide, acyl carrier protein 65-74, was synthesized using a Milligen/Biosearch model 9600 peptide synthesizer, with the standard BOP + HOBt coupling programs (Milligen/Biosearch. Novato, CA). In consecutive runs, Pepsyn K™, normal polystyrene and Tentagel™ supports were also used to make the same sequence. The resulting peptides were analyzed by amino acid analysis (AAA), high performance liquid chromatography (HPLC) and fast atom bombardment (FAB) mass spectrometry. The results showed that synthesis using PEG-PS provided the product in the highest yield with superior purity.

## Claims (Claims for the following Contracting State(s): BE, CH, LI, DE, DK, FR, GB, IT, NL, SE)

1. A resin for solid-phase peptide synthesis having the formula: 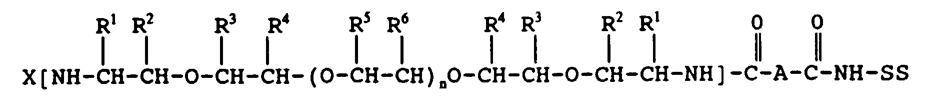 wherein n is an integer from 5 to 150; R1, R², R³, R⁴, R⁵ and R⁶ are independently selected from the group consisting of H, alkyl groups and aryl groups; X is H or H₂N-B-NH-C(O)-A-C(O)-; A and B are independently selected from the group consisting of straight chain or branched alkyl groups having up to 10 carbon atoms, CH=CH groups and aromatic groups; and SS is a solid support, wherein the ω-amino group is optionally protected by an N^{ω} protecting group.

2. A resin of Claim 1, wherein R¹, R², R³, R⁴, R⁵ and R⁶ are independently H, methyl groups or ethyl groups.

3. A resin of Claim 1, wherein A is selected from the group consisting of ethylene, propylene, isopropylene, butylene and isobutylene.

4. A resin of any of Claims 1-3, wherein the solid support is selected from the group consisting of amino-functionalized membranes, amino-functionalized porous glass, amino-functionalized silica, amino-functionalized polystyrenes, amino-functionalized polydimethylacrylamides, amino-functionalized cotton and amino-functionalized paper.

5. A resin of Claim 4, wherein the polystyrene is selected from the group consisting of amino-polystyrene, aminomethyl polystyrene, aminoacyl polystyrene and p-methylbenzhydrylamine polystyrene.

## Claims (Claims for the following Contracting State(s): ES)

1. A method for making a support for solid-phase peptide synthesis comprising making the support from a resin having the formula: 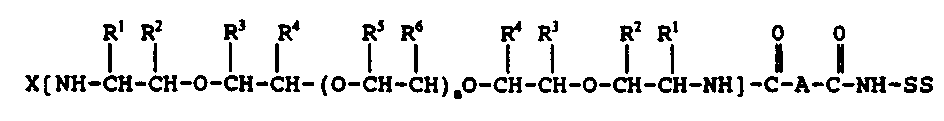 wherein n is an integer from 5 to 150; R1, R², R³, R⁴, R⁵ and R⁶ are independently selected from the group consisting of H, alkyl groups and aryl groups; X is H or H₂N-B-NH-C(O)-A-C(O)-; A and B are independently selected from the group consisting of straight chain or branched alkyl groups having up to 10 carbon atoms, CH=CH groups and aromatic groups; and SS is a solid support, wherein the ω-amino group is optionally protected by an N^{ω} protecting group.

2. A method of Claim 1, wherein R¹, R², R³, R⁴, R⁵ and R⁶ are independently H, methyl groups or ethyl groups.

3. A method of Claim 1, wherein A is selected from the group consisting of ethylene, propylene, isopropylene, butylene and isobutylene.

4. A method of any of Claims 1-3, wherein the solid support is selected from the group consisting of amino-functionalized membranes, amino-functionalized porous glass, amino-functionalized silica, amino-functionalized polystyrenes, amino-functionalized polydimethylacrylamides, amino-functionalized cotton and amino-functionalized paper.

5. A method of Claim 4, wherein the polystyrene is selected from the group consisting of amino-polystyrene, aminomethyl polystyrene, aminoacyl polystyrene and p-methylbenzhydrylamine polystyrene.

## Patentansprüche (Patentansprüche für folgende(n) Vertragsstaat(en): BE, CH, LI, DE, DK, FR, GB, IT, NL, SE)

1. Harz für die Festphasen-Peptidsynthese, das die Formel 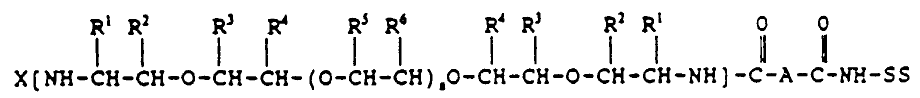 aufweist, in der bedeuten:
- n eine ganze Zahl von 5 bis 150,
- R¹, R², R³, R⁴, R⁵ und R⁶ Gruppen, die unabhängig voneinander unter Wasserstoff, Alkylgruppen und Arylgruppen ausgewählt sind,
- X Wasserstoff oder H₂N-B-NH-C(O)-A-C(O)-,
- A und B Gruppen, die unabhängig voneinander unter geradkettigen oder verzweigten Alkylgruppen mit bis zu 10 Kohlenstoffatomen, Gruppen, die -CH=CH- enthalten, und aromatischen Gruppen ausgewählt sind, und
- SS einen festen Träger,
wobei die ω-Aminogruppe durch eine N^{ω}-Schutzgruppe geschützt ist.

2. Harz nach Anspruch 1, wobei R¹, R², R³, R⁴, R⁵ und R⁶ unabhängig Wasserstoff, Methylgruppen oder Ethylgruppen darstellen.

3. Harz nach Anspruch 1, wobei A unter Ethylen, Propylen, Isopropylen, Butylen und Isobutylen ausgewählt ist.

4. Harz nach einem der Ansprüche 1 bis 3, wobei der feste Träger unter aminofunktionalisierten Membranen, aminofunktionalisiertem porösem Glas, aminofunktionalisiertem Siliciumdioxid, aminofunktionalisierten Polystyrolen, aminofunktionalisierten Polydimethylacrylamiden, aminofunktionalisierter Baumwolle und aminofunktionalisiertem Papier ausgewählt ist.

5. Harz nach Anspruch 4, wobei das Polystyrol unter Aminopolystyrolen, Aminomethylpolystyrolen, Aminoacylpolystyrolen und p-Methylbenzhydrylaminpolystyrolen ausgewählt ist.

## Patentansprüche (Patentansprüche für folgende(n) Vertragsstaat(en): ES)

1. Verfahren zur Herstellung eines Trägers für die Festphasen-Peptidsynthese, das die Herstellung des Trägers aus einem Harz der Formel 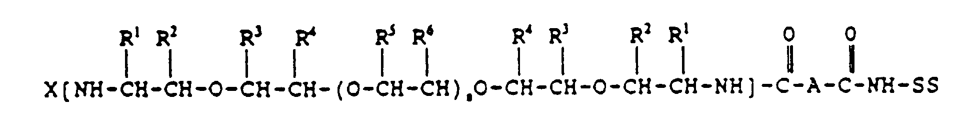 umfaßt, in der bedeuten:
- n eine ganze Zahl von 5 bis 150,
- R¹, R², R³, R⁴, R⁵ und R⁶ Gruppen, die unabhängig voneinander unter Wasserstoff, Alkylgruppen und Arylgruppen ausgewählt sind,
- X Wasserstoff oder H₂N-B-NH-C(O)-A-C(O)-,
- A und B Gruppen, die unabhängig voneinander unter geradkettigen oder verzweigten Alkylgruppen mit bis zu 10 Kohlenstoffatomen, Gruppen, die -CH=CH- enthalten, und aromatischen Gruppen ausgewählt sind, und
- SS einen festen Träger,
wobei die ω-Aminogruppe durch eine N^{ω}-Schutzgruppe geschützt ist.

2. Verfahren nach Anspruch 1, wobei R¹, R², R³, R⁴, R⁵ und R⁶ unabhängig Wasserstoff, Methylgruppen oder Ethylgruppen darstellen.

3. Verfahren nach Anspruch 1, wobei A unter Ethylen, Propylen, Isopropylen, Butylen und Isobutylen ausgewählt ist.

4. Verfahren nach einem der Ansprüche 1 bis 3, wobei der feste Träger unter aminofunktionalisierten Membranen, aminofunktionalisiertem porösem Glas, aminofunktionalisiertem Siliciumdioxid, aminofunktionalisierten Polystyrolen, aminofunktionalisierten Polydimethylacrylamiden, aminofunktionalisierter Baumwolle und aminofunktionalisiertem Papier ausgewählt ist.

5. Verfahren nach Anspruch 4, wobei das Polystyrol unter Aminopolystyrolen, Aminomethylpolystyrolen, Aminoacylpolystyrolen und p-Methylbenzhydrylaminpolystyrolen ausgewählt ist.

## Revendications (Revendications pour l'(les) Etat(s) contractant(s) suivant(s): BE, CH, LI, DE, DK, FR, GB, IT, NL, SE)

1. Résine pour la synthèse peptidique en phase solide, ayant la formule : 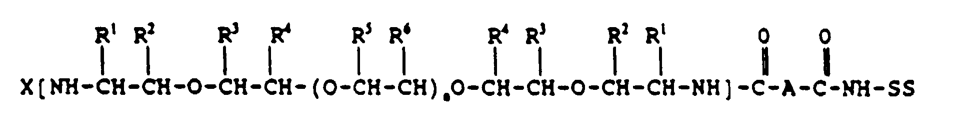 dans laquelle
n est un entier de 5 à 150 ; R¹, R², R³, R⁴, R⁵ et R⁶ sont indépendamment choisis parmi un atome d'hydrogène, des groupes alkyle et des groupes aryle ;
X représente H ou H₂N-B-NH-C(O)-A-C(O)- ; A et B sont indépendamment choisis parmi des groupes alkyle à chaîne droite ou ramifiée ayant jusqu'à 10 atomes de carbone,
des groupes CH=CH et des groupes aromatiques ; et SS est un support solide,
dans laquelle le groupe amino situé en ω est éventuellement protégé par un groupe protecteur de N^{ω}.

2. Résine selon la revendication 1, dans laquelle R¹, R², R³, R⁴, R⁵ et R⁶ sont indépendamment un atome d'hydrogène, un groupe méthyle, ou un groupe éthyle.

3. Résine selon la revendication 1, dans laquelle A est choisi parmi l'éthylène, le propylène, l'isopropylène, le butylène et l'isobutylène.

4. Résine selon l'une quelconque des revendications 1 à 3, dans laquelle le support solide est choisi parmi des membranes fonctionnalisées par des groupes amino, du verre poreux fonctionnalisé par des groupes amino, de la silice fonctionnalisée par des groupes amino, des polystyrènes fonctionnalisés par des groupes amino, des polydiméthylacrylamides fonctionnalisés par des groupes amino, du coton fonctionnalisé par des groupes amino et du papier fonctionnalisé par des groupes amino.

5. Résine selon la revendication 4, dans laquelle le polystyrène est choisi parmi de l'aminopolystyrène, de l'aminométhyl polystyrène, de l'aminoacyl polystyrène et du p-méthylbenzhydrylamine polystyrène.

## Revendications (Revendications pour l'(les) Etat(s) contractant(s) suivant(s): ES)

1. Procédé de fabrication d'un support pour pour la synthèse peptidique en phase solide, comprenant la réalisation du support à partir d'une résine ayant la formule : 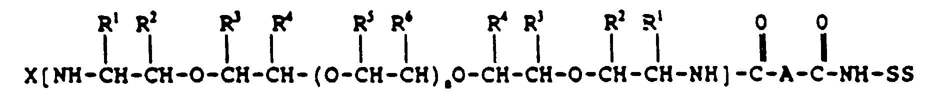
dans laquelle n est un entier de 5 à 150 ; R¹, R², R³, R⁴, R⁵ et R⁶ sont indépendamment choisis parmi un atome d'hydrogène, des groupes alkyles et des groupes aryles ;
X représente H ou H₂N-B-NH-C(O)-A-C(O)- ; A et B sont indépendamment choisis parmi des groupes alkyles à chaîne droite ou ramifiée ayant jusqu'à 10 atomes de carbone,
des groupes CH=CH et des groupes aromatiques ; et SS est un support solide,
dans laquelle le groupe amino situé en ω est éventuellement protégé par un groupe protecteur de N^{ω}.

2. Procédé selon la revendication 1, dans lequel R¹, R², R³, R⁴, R⁵ et R⁶ sont indépendamment un atome d'hydrogène, un groupe méthyle, ou un groupe éthyle.

3. Procédé selon la revendication 1, dans lequel A est choisi parmi l'éthylène, le propylène, l'isopropylène, le butylène et l'isobutylène.

4. Procédé selon l'une quelconque des revendications 1 à 3, dans lequel le support solide est choisi parmi des membranes fonctionnalisées par des groupes amino, du verre poreux fonctionnalisé par des groupes amino, de la silice fonctionnalisée par des groupes amino, des polystyrènes fonctionnalisés par des groupes amino, des polydiméthylacrylamides fonctionnalisés par des groupes amino, du coton fonctionnalisé par des groupes amino et du papier fonctionnalisé par des groupes amino.

5. Procédé selon la revendication 4, dans lequel le polystyrène est choisi parmi de l'aminopolystyrène, de l'aminométhyl polystyrène, de l'aminoacyl polystyrène et du p-méthylbenzhydrylamine polystyrène.
